# NEUE EUROPÄISCHE PATENTSCHRIFT

(11) **EP 2 471 501 B2**
(45) Veröffentlichungstag und Bekanntmachung des Hinweises auf die Entscheidung über den Einspruch: **17.11.2021**
(45) Hinweis auf die Patenterteilung: 28.08.2013
(21) Anmeldenummer: 11008456.3
(22) Anmeldetag: 11.09.2009
(51) Int. Cl.: A61K 8/06, A61K 8/35, A61K 8/40, A61K 8/49, A61K 8/97, A61Q 17/04

(54) **UV-Filter haltige O/W-Wirkstoffemulsion**
Oil/water active ingredient emulsion containing UV filter
Émulsion de substance active huile dans eau contenant un filtre UV

(30) Priorität: 16.09.2008 DE 102008048328
(43) Veröffentlichungstag der Anmeldung: 04.07.2012
(62) Teilanmeldung aus: 09778483.9
(73) Patentinhaber: Beiersdorf AG, 20253 Hamburg (DE)
(72) Erfinder: Ruppert, Stephan, Dr., 20259 Hamburg (DE); Blohm, Alexandra, 20257 Hamburg (DE); Steikert, Claudia, 20255 Hamburg (DE)
(74) Vertreter: Beiersdorf AG

(56) Entgegenhaltungen:
- EP-A1- 1 566 172
- DE-A1- 10 356 164
- DE-A1- 10 356 164
- DE-A1- 10 356 187
- DE-A1- 10 356 187
- US-A1- 2007 196 289
- NEUES AUS DERMATOLOGIE, KOSMETIK UND WUNDVERSORGUNG - BEITRÄGE ZUM STAND DER TECHNIK, Nr. 18, Oktober 2007 (2007-10), XP040423227,
- DATABASE WPI Section Ch, Week 200478 Thomson Scientific, London, GB; Class B04, AN 2004-785480 XP002563281, "External use composition containing oil-soluble licorice extract and its stabilization method", & CN 1 520 811 A (MARUZEN PHARMA CO LTD) 18. August 2004 (2004-08-18)
- CHEMICAL ABSTRACTS, Bd. 54, Nr. 2, 16. November 1998 (1998-11-16), Columbus, Ohio, US; abstract no.: 142:43426, CHO, HWAN ILL ET AL.: "Liquid crystal gel for stabilized oil soluble extracts of glycyrrhizae radix and cosmetic compositions", XP002563280 & KR 0 154 366 B1 (LG CHEMICAL LTD [KR]) 16. November 1998 (1998-11-16)
- Beiersdorf AG: "Vergleichsversuche", European Patent Register , 21. Oktober 2011 (2011-10-21), XP002672026, Gefunden im Internet: URL:https://register.epo.org/espacenet/app lication?number=EP09778483&lng=en&tab=docl ist [gefunden am 2011-10-21]

## Beschreibung

Die vorliegende Erfindung betrifft eine kosmetische O/W-Emulsion enthaltend
a) 2-Ethythexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Süßholzextrakt
d) 2,4-Bis-{[4-(2-ethyl-hexyloxy)-2-hydroxy]-phenyl}-6-(4-methoxyphenyl)-1,3,5-triazin (INCI: Aniso Triazin), dadurch gekennzeichnet,dass als O/W-Emulgator Glycerylstearatcitrat, Glycerylstearat SE, Stearinsäure, Polyglyceryl-3-methylglucosedistearat, PEG-40 Stearat, PEG-100 Stearat, Kaliumcetylphosphat und/oder eine Mischung aus Cetearylalkohol + PEG-40 hydriertes Rizinusöl + Natriumcetearylsulfat + Glycerylstearat eingesetzt wird, sowie eine kosmetische O/W-Emulsion enthaltend
   a) 2-Ethythexyl-2-cyano-3,3-diphenylacrylat (INCI: Octocrylene),
   b) 4-(tert.-Butyl)-4'-methoxydibenzoylmethan (INCI: Butylmethoxydibenzoylmethan),
   c) Licochalcon A,
   d) 2,4-Bis-{[4-(2-ethyl-hexyloxy)-2-hydroxy]-phenyl}-6-(4-methoxyphenyl)-1,3,5-triazin (INCI: Aniso Triazin).

Die Haut ist das größte Organ des Menschen. Sie hat eine Vielzahl lebenswichtiger Funktionen zu erfüllen, beispielsweise die Wärmeregulation und die Barrierefunktion gegen das Austrocknen der Haut und des gesamten Organismus sowie als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Haut ist einer Vielzahl an physikalischen, chemischen und biologischen Belastungen ausgesetzt. Eine Vielzahl dieser Belastungen führt aus unterschiedlichen Gründen zu einer vorübergehenden oder dauerhaften Rötung der Haut.

Damit die Haut ihre biologischen Funktionen erfüllen kann, bedarf sie der regelmäßigen Reinigung und Pflege. Um die Regeneration der Haut zu fördern, sie vor einer vorzeitigen Alterung zu schützen oder Reizungen zu vermeiden werden kosmetischen Hautpflegeprodukten in der Regel Wirkstoffe zugesetzt.

Zu den in der Hautpflege eingesetzten Wirkstoffen gehören beispielsweise Süßholzextrakte. Der Schlüsselwirkstoff von Süßholzextrakten, insbesondere von *Glycyrrhiza inflata*, ist dabei die Verbindung Licochalcon A, welche die folgende Struktur aufweist:

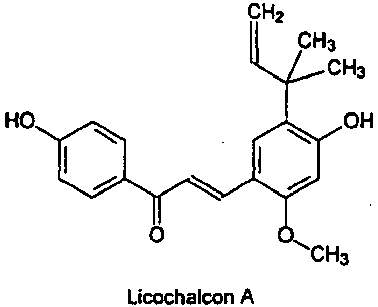

Licochalcon A enthaltende Süßholzextrakte werden dabei insbesondere gegen Hautreizungen wie Rötungen eingesetzt.

Nachteilig am Stande der Technik von Süßholzextrakten enthaltenden kosmetischen Zubereitungen (insbesondere, wenn diese Extrakte Licochalcon A enthalten), ist jedoch der Umstand, dass die Extrakte und insbesondere das Licochalcon A nicht besonders lagerstabil sind. Werden diese Zubereitungen über einen längeren Zeitraum und insbesondere bei höheren Temperaturen gelagert, kommt es zu einem Abbau des/der Wirkstoffe in der Zubereitung und das Kosmetikum verliert langsam an Wirksamkeit. Dieser Wrksamkeitsverlust tritt besonders stark bei O/W-Emulsionen (Öl-in-Wasser-Emulsionen) auf.

Es war daher die Aufgabe der vorliegenden Erfindung, die Nachteile des Standes der Technik zu beseitigen und lagerstabile bzw. temperaturstabile kosmetische Zubereitungen zu entwickeln, die Süßholzextrakte bzw. Licochalcon A enthalten.

Überraschend gelöst wird die Aufgabe durch eine kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Süßholzextrakt und
d) 2,4-Bis-{[4-(2-ethyl-hexyloxy)-2-hydroxy]-phenyl}-6-(4-methoxyphenyl)-1,3,5-triazin (INCI: Aniso Triazin).
dadurch gekennzeichnet,dass als O/W-Emulgator Glycerylstearatcitrat, Glycerylstearat SE, Stearinsäure, Polyglyceryl-3-methylglucosedistearat, PEG-40 Stearat, PEG-100 Stearat, Kaliumcetylphosphat und/oder eine Mischung aus Cetearylalkohol + PEG-40 hydriertes Rizinusöl + Natriumcetearylsulfat + Glycerylstearat eingesetzt wird.

Die Aufgabe wird auch überraschend gelöst durch eine kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Licochalcon A und
d) 2,4-Bis-{[4-(2-ethyl-hexyloxy)-2-hydroxy]-phenyl-6-(4-methoxyphenyl)-1,3,5-triazin (INCI: Aniso Triazin).

Zwar kennt der Fachmann an sich kosmetische Zubereitungen mit Süßholzextrakten und insbesondere mit Licochalcon A. So beschreiben die deutschen Offenlegungsschriften DE 102 24 387.5, DE 103 52 368.5, DE 103 52 367.7, DE 103 56 723.2, DE 103 56175.7, DE 103 42 212.9, DE 103 52 369.3, DE 103 56 187.0, DE 103 57 451.4, DE 103 57 452.2, DE 103 56 164.1, DE 103 56 870.0, DE 103 56 869.7 und DE 103 56 866.2 Licochalcon A enthaltende Zubereitungen. Diese Schriften konnten jedoch nicht den Weg zur vorliegenden Erfindung weisen, da in den offenbarten Rezepturen entweder einer der für die Erfindung erforderlichen UV-Filter fehlt oder die Zusammensetzungen in einer anderen Grundlage (insbesondere W/O-Emulsion) vorliegen. Darüber hinaus kennt der Stand der Technik "Neues aus Dermatologie, Kosmetik und Wundversorgung-Beiträge zum Stand der Technik, Nr. 18, Oktober 2007, die WPI Database Einträge XP-002563281, XP-002563280, die EP 1566172, das International Cosmetic Ingredient Dictionary and Handbook, 11th edition, volume 1, 2006, Seite 252 sowie "the Chemistry and Manufacture of Cosmetics", Volume II-Formulating, 2009, Seiten 73-99, doch konnten diese Schriften nicht den Weg zur vorliegenden Erfindung weisen.

Der erfindungsgemäß bevorzugte Süßholzextrakt stammt aus der Pflanze *Glycyrrhiza inflata.*

Erfindungsgemäß ist ferner vorteilhaft, wenn der Extrakt in Form eines wäßrigen Auszugs vorliegt, in welchem
- Licochalkon A
- Wasser
- gegebenenfalls ein oder mehrere Polyole
vorliegen.

Erfindungsgemäß vorteilhafte Ausführungsformen der vorliegenden Erfindung dadurch gekennzeichnet, dass die Zubereitung mindestens 2,1 Gew.%, bezogen auf das Gesamtgewicht der Zubereitung, an 4-(tert.-Butyl)-4'-methoxydibenzoylmethan enthält.

Es ist generell erfindungsgemäß bevorzugt, die erfindungsgemäßen Bestandteile in den folgenden Konzentrationen einzusetzen:
a) 2-Ethylhexyl-2-cyano-3,3-diphenylacrylat (INCI: Octocrylene) in einer Konzentration von 0,1 bis 12 Gewichts- %, bezogen auf das Gesamtgewicht der Zubereitung.
b) 4-(tert.-Butyl)-4'-methoxydibenzoylmethan (INCI: Butylmethoxydibenzoylmethan) in einer Konzentration von 0,1 bis 8 Gewichts-%, bezogen auf das Gesamtgewicht der Zubereitung.
c) Süßholzextrakt in einer Konzentration von 0,001 bis 0,05 Gewichts-%, bezogen auf das Gesamtgewicht der Zubereitung.
d) Licochalcon A in einer Konzentration von 0,0001 bis 0,01 Gewichts-%, bezogen auf das Gesamtgewicht der Zubereitung.

2,4-Bis-{[4-(2-ethyl-hexyloxy)-2-hydroxy]-phenyl}-6-(4-methoxyphenyl)-1,3,5-triazin wird erfindungsgemäß vorteilhaft in einer Konzentration 0,1 von bis 5 Gewichts-%, bezogen auf das Gesamtgewicht der Zubereitung, eingesetzt.

Es ist erfindungsgemäß vorteilhaft, wenn als O/W-Emulgator Glycerylstearatcitrat, Glycerylstearat SE, Stearinsäure, Polyglyceryl-3-methylglucosedistearat, PEG-40 Stearat, PEG-100 Stearat, Kaliumcetylphosphat und/oder eine Mischung aus Cetearylalkohol + PEG-40 hydriertes Rizinusöl + Natriumcetearylsulfat + Glycerylstearat, Natrium Stearoyl Glutamat, Sucrose Polystearate + Hydrogenated Polyisobuten Polyglyceryl-3 Methylglucose Distearate Triceteareth-4 Phosphate eingesetzt wird, wenn Licochalkon A als Substanz c) eingesetzt wird.

Erfindungsgemäß vorteilhafte Ausführungsformen sind auch dadurch gekennzeichnet, dass die Zubereitung einen oder mehrere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden) campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl) ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Co-polymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch:2,4,6-Tris-[anilino-(p-carbo-2'-ethyl-1'-hexyloxy)]-1,3,5-triazin(INCI: Ethylhexyl Triazone); 2,4-Bis-(4'-Di-neopentylaminobenzalmalonat)-6-(4"-butylaminobenzoat)-s-triazin, Titandioxid; Zinkoxid, Trisbiphenyl-Triazin 4-Dicyanomethylen-2,6-dimethyl-1,4-dihydropyridin-N-(ethyloxysulfatestersalz) das mono-Natriumsalz, enthält.

Es ist erfindungsgemäß vorteilhaft, wenn die erfindungsgemäße Zubereitung frei ist von p-Methylbenzylidencampher.

Es ist erfindungsgemäß bevorzugt, wenn die erfindungsgemäße Zubereitung Ethanol enthält. Darüber hinaus kann die erfindungsgemäße Zubereitung vorteilhafter Weise ein oder mehrere weitere Verbindungen mit Alkoholfunktion enthalten, beispielsweise Glycerin, 2-Methyl-1,3-propandiol, Pentan-1,2-diol, Hexan-1,2-diol, Heptan-1,2-diol, Octan-1,2-diol, Nonan-1,2-diol, Decan-1,2-diol, Propanole, Propan- und Butandiole.

Es ist erfindungsgemäß vorteilhaft, wenn die erfindungsgemäße Zubereitung ein oder mehrere Antioxidantien enthält.

Es ist erfindungsgemäß bevorzugt, wenn die Zubereitung eine oder mehrere Verbindungen gewählt aus der Gruppe der Verbindungen Tocopherol, Tocopherolacetat, 2,6-Di-tert-butyl-4-methylphenol enthält.

Die erfindungsgemäße Zubereitung kann darüber hinaus ein oder mehrere weitere Wirkstoffe enthalten, beispielsweise alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Niacinamid, Vitamin A bzw. seine Derivate.

Es ist erfindungsgemäß vorteilhaft, wenn die Zubereitung ein oder mehrere Polymere enthält. Erfindungsgemäß bevorzugte Ausführungsformen sind dadurch gekennzeichnet, dass die Zubereitung ein oder mehrere Polymere gewählt aus der Gruppe der Verbindungen Acrylat/C10-C30 Alkylacrylat Crosspolymer, Acrylate, Carbomere, Tapiokastärke, Xanthangummi enthält.

Es ist vorteilhaft im Sinne der vorliegenden Erfindung, wenn die Ölphase der O/W-Emulsion ein oder mehrere Ölkomponenten gewählt aus der Gruppe der Verbindungen Butylenglycol Dicaprylat/Dicaprate, Myristylmyristat, Octyldodecanol, C12-15 Alkyl Benzoat Caprylic/Capric Triglyceride, Diisopropyl Sebacate, Dicaprylyl Ether, Mineralöl, Silikonöl enthält.

Es ist erfindungsgemäß vorteilhaft, wenn die erfindungsgemäße Zubereitung ein oder mehrere Konservierungsmittel, insbesondere ein oder mehrere Parabene und/oder Phenoxyethanol enthält.

Darüber hinaus kann die erfindungsgemäße O/W-Emulsion weitere kosmetischen Wirk-, Hilfsund Zusatzstoffe enthalten, beispielsweise EDTA und Parfümstoffe wie Limonene, Linalool, Benzyl Benzoate, Hydroxyisohexyl 3-Cyclohexene Carboxaldehyde, Hexyl Cinnamal, Benzyl Salicylate, Eugenol, Butylphenyl Methylpropional, Alpha-Isomethyl lonone, Citronellol, Coumarin, Geraniol, Cinnamyl Alcohol, Citral

Ferner können in der Emulsion Sensorikadditive wie Distarch Phosphate Natrium Stärke Octenylsuccinate, Alumiunium Stärke Octenylsuccinate, Acrylonitrile-methacrylonitrile-methylmethacrylate Copolymer + Isopentane + Magnesium Hydroxide, Tapioca Stärke, Silica, Nylon-6 Polyamide-5, Talkum mikron

Das erfindungsgemäße Herstellungsverfahren und das durch das Herstellungsverfahren hergestellte Produkt sind erfindungsgemäß durch die folgenden Details als erfindungsgemäß vorteilhaft gekennzeichnet:

So ist es erfindungsgemäß vorteilhaft, wenn der oder die Emulgatoren der Ölphase zugesetzt wurden bevor diese zur wässrigen Phase zugefügt wird.

Die heiße Wasserphase weist erfindungsgemäß vorteilhaft eine Temperatur von 50 bis 95 °C, bevorzugt eine Temperatur von 75 bis 90 °C und besonders bevorzugt eine Temperatur von etwa 85 °C auf. Der Begriff "etwa" soll dabei alleine die für derartige Verfahren typischen leichten Temperaturschwankungen kennzeichnen.

Die erhitzte Ölphase weist erfindungsgemäß vorteilhaft eine Temperatur von 50 bis 95 °C, bevorzugt eine Temperatur von 75 bis 90 °C und besonders bevorzugt eine Temperatur von etwa 85 °C auf. Der Begriff "etwa" soll dabei alleine die für derartige Verfahren typischen leichten Temperaturschwankungen kennzeichnen.

Als Homogenisatoren werden erfindungsgemäß vorteilhaft Mischer der Firma Becomix oder Krieger verwendet.

Die Homogenisierung erfolgt erfindungsgemäß vorteilhaft in einem Zeitraum von 2 bis 25 Minuten.

Die Rührgeschwindigkeit bei der Homogenisierung beträgt erfindungsgemäß vorteilhaft von 500 bis 2000 U/min., wobei eine Rührgeschwindigkeit von 1000 bis 1400 U/min. erfindungsgemäß bevorzugt, und eine Rührgeschwindigkeit von 1200 U/min (+-50 U/min.) erfindungsgemäß besonders bevorzugt ist.

Im Verfahrensschritt d) wird die Zubereitung erfindungsgemäß vorteilhaft auf eine Temperatur von 25 bis 40°C abgekühlt, wobei eine Temperatur von etwa 35 °C bevorzugt ist. Der Begriff "etwa" soll dabei alleine die für derartige Verfahren typischen leichten Temperaturschwankungen kennzeichnen.

Die Parfümstoffe und Konservierungsmittel werden vor der Zugabe zur Emulsion bevorzugt miteinander vermischt.

Die Parfümstoffe und Konservierungsmittel werden vor der Zugabe zur Emulsion bevorzugt in Ethanol gelöst.

Die Zugabe der Parfümstoffe und Konservierungsmittel erfolgt erfindungsgemäß vorteilhaft bei einer Temperatur von unter 30 °C.

Vorteilhaft im Sinne der vorliegenden Erfindung können die Zubereitungen zur Pflege der Haut, dem kosmetischen Lichtschutz oder als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcreme, Tages- oder Nachtcräme usw. Es ist gegebenenfalls möglich und vorteilhaft, die erfindungsgemäßen Zusammensetzungen als Grundlage für pharmazeutische Formulierungen zu verwenden.

Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

### Vergleichsversuche

Mit den folgenden Vergleichsversuchen konnte der erfinderische Effekt gezeigt werden:
Es wurden die folgenden Rezepturen hergestellt und der Gehalt an Süßholzextrakt (enthaltend Licochalcon A) vor und nach einer 4 wöchigen Lagerung in einem Brutschrank bei 40 °C bestimmt.

Die Rezeptur Muster 1 enthält eine Zubereitung ohne die Stabilisatoren Butylmethoxydibenzoylmethan und Octocrylen. Die Rezeptur Muster 2 enthält zusätzlich die beiden Stabilisatoren.

| | **Muster 1** | **Muster 2** |
|---|---|---|
| **INCI-Name(n)** | **m [%]** | **m [%]** |
| Alcohol Denat. | 6,00 | 6,00 |
| Natrium Hydroxid | 0,60 | 0,60 |
| Cetearyl Alcohol + PEG-40 Castor Oil + Natrium Cetearyl Sulfat | 2,00 | 2,00 |
| Glyceryl Stearat SE | 0,80 | 0,80 |
| Trinatrium EDTA | 1,00 | 1,00 |
| Methylparaben | 0,30 | 0,30 |
| Phenoxyethanol | 0,20 | 0,20 |
| Bis-Ethylhexyloxyphenol Methoxyphenyl Triazin | 3,50 | 3,50 |
| **Butyl Methoxydibenzoylmethan** | | **4,50** |
| Diethylhexyl Butamido Triazon | 1,00 | 1,00 |
| Ethylhexyl Methoxycinnamat + BHT | 2,00 | 2,00 |
| **Octocrylene** | | **4,50** |
| Phenylbenzimidazol Sulfonsäure | 2,00 | 2,00 |
| Glycerin | 2,00 | 2,00 |
| Butylene Glycol Dicaprylate/Dicaprat | 7,00 | 7,00 |
| C12-15 Alkyl Benzoat | 11,00 | 11,00 |
| Myristyl Myristat | 2,00 | 2,00 |
| Octyldodecanol | 5,50 | 5,50 |
| Glycyrrhiza Inflata Root Extract (Licochalcon A) | 0,025 | 0,025 |
| Titandioxid + Trimethoxycaprylylsilan | 4,00 | 4,00 |
| Acrylates/C10-30 Alkyl Acrylat Crosspolymer | 0,05 | 0,05 |
| Cetyl Alcohol | 2,50 | 2,50 |
| Tapioca Starch | 1,00 | 1,00 |
| Xanthan Gum | 0,80 | 0,80 |
| Tocopheryl Acetat | 0,50 | 0,50 |
| Wasser | ad 100,000 | ad 100,000 |

### Versuchsdesign:

Bestimmung des Süßholzgehaltes (Licochalcon A) des Prüfmusters nach 4 Wochen Lagerstabilität bei 40°C Brutschrank Lagerung.

### Eingesetzte Methode:

Der Süßholzgehalt wurde mittels HPLC-DAD über eine externe Kalibrierreihe ermittelt. Zur Kalibrierung wurde das von der Auftraggeberin bereitgestellte Süßholz, NART: 96146-90000-00, Lot 17743901 eingesetzt. Die im Bericht angegebenen Gehalte beziehen sich auf die zur Kalibrierung eingesetzte Referenzsubstanz.

### Ergebnisse:

| **Probenbezeichnung** | **Gehalt in %** | |
|---|---|---|
| | **ist** | **Soll** |
| Muster 1 | **< 0,005** | **0,025** |
| Muster 2 | 0,018 | 0,025 |

### Fazit:

Die Probe Muster 2 enthält mehr Süßholzextrakt (Licochalcon A) als die Probe Muster 1. Der Zusatz der Stabilisatoren Butylmethoxydibenzoylmethan und Octocrylen führt zu einer erhöhten Temperatur- und Lagerstabilität beim Süßholzextrakt (Licochalcon A).

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

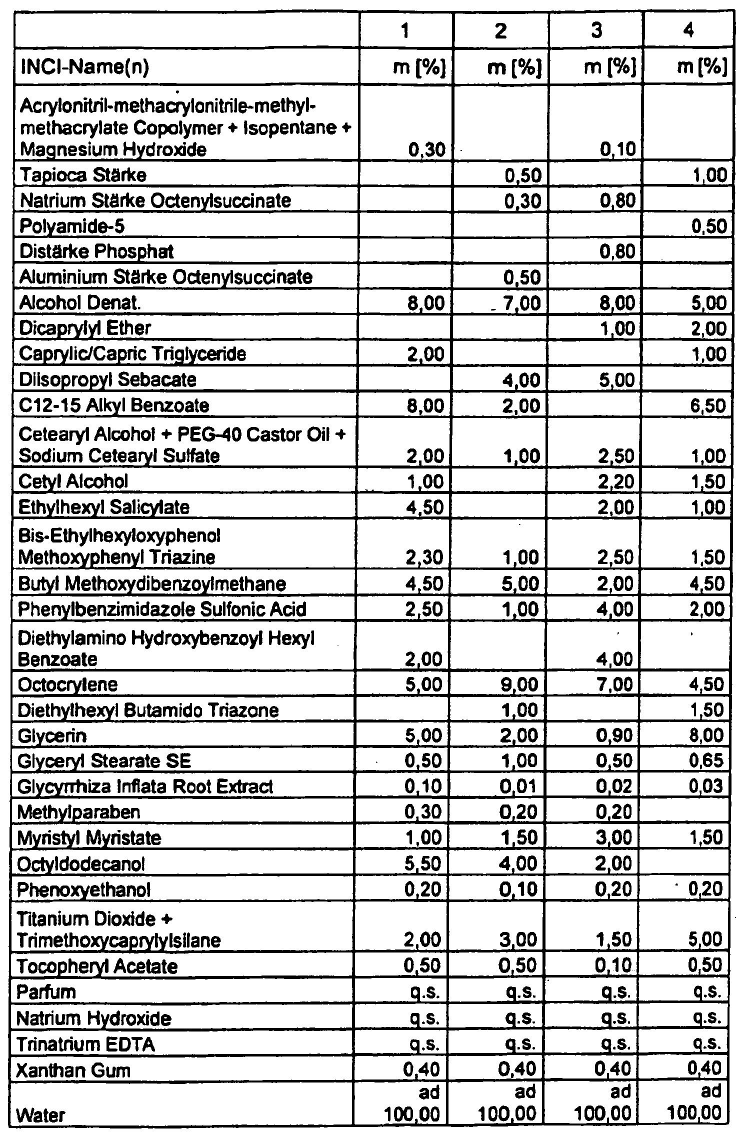

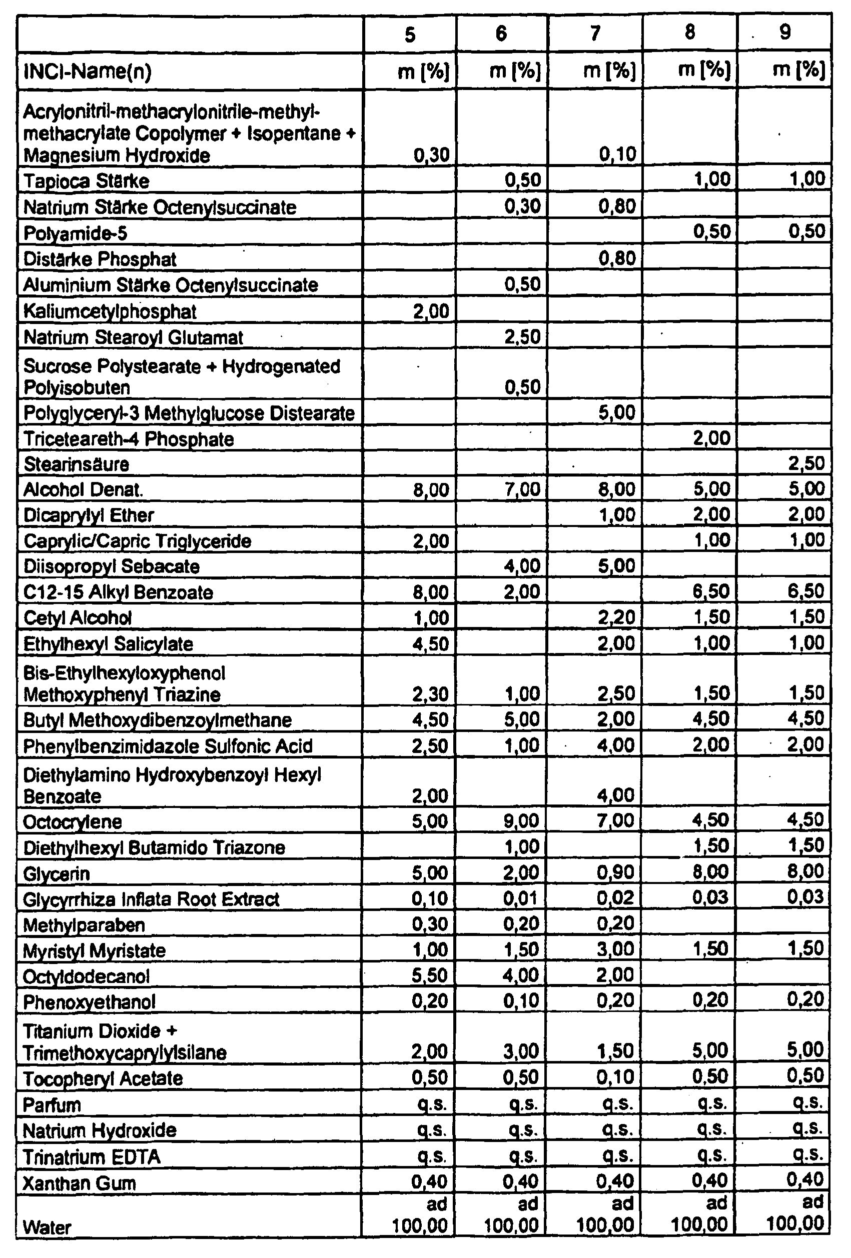

## Patentansprüche

1. Kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Süßholzextrakt und
d) 2,4-Bis-{[4-(2-ethyl-hexyloxy)-2-hydroxy)-phenyl}-6-(4-methoxyphenyl)-1,3,5-triazin (INCI: Aniso Triazin), **dadurch gekennzeichnet, dass** als O/W-Emulgator Glycerylstearatcitrat, Glycerylstearat SE, Stearinsäure, Polyglyceryl-3-methylglucosedistearat, PEG-40 Stearat, PEG-100 Stearat, Kaliumcetylphosphat und/oder eine Mischung aus Cetearylalkohol + PEG-40 hydriertes Rizinusöl + Natriumcetearylsulfat + Glycerylstearat eingesetzt wird.

2. Kosmetische O/W-Emulsion enthaltend
a) 2-Ethylhexyl-2-cyano-3,3-diphenylacrylat (INCI: Octocrylene),
b) 4-(tert.-Butyl)-4'-methoxydibenzoylmethan (INCI: Butylmethoxydibenzoylmethan),
c) Licochalcon A und
d) 2,4-Bis-{[4-(2-ethyl-hexyloxy)-2-hydroxy]-phenyl}-6-(4-methoxyphenyl)-1,3,5-triazin (INCI: Aniso Triazin).

3. Kosmetische O/W-Emulsion nach einem der vorhergehenden Ansprüche, **dadurch gekennzeichnet, dass** die Zubereitung mindestens 2,1 Gew.%, bezogen auf das Gesamtgewicht der Zubereitung, an 4-(tert.-Butyl)-4'-methoxydibenzoylmethan enthält.

4. Kosmetische O/W-Emulsion nach Anspruch 2, **dadurch gekennzeichnet, dass** als O/W-Emulgator Glycerylstearatcitrat, Glycerylstearat SE, Stearinsäure, Polyglyceryl-3-methylglucosedistearat, PEG-40 Stearat, PEG-100 Stearat, Kaliumcetylphosphat und/oder eine Mischung aus Cetearylalkohol + PEG-40 hydriertes Rizinusöl + Natriumcetearylsulfat + Glycerylstearat eingesetzt wird.

5. Kosmetische O/W-Emulsion nach einem der vorhergehenden Ansprüche, **dadurch gekennzeichnet, dass** die Zubereitung einen oder mehrere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat;
Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, Homomenthylsalicylat; 2-Ethylhexyl-2-hydroxybenzoat; Dimethicodiethylbenzalmaloriat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-(4'- Di-neopentylaminobenzalmalonat)-6-(4"-butylaminobenzoat)-s-triazin, Titandioxid; Zinkoxid, Trisbiphenyl-Triazin 4-Dicyanomethylen-2,6-dimethyl-1,4-dihydropyridin-N-(ethyloxysulfatestersalz) das mono-Natriumsalz, enthält.

6. Kosmetische O/W-Emulsion, Verwendung oder Verfahren nach einem der vorhergehenden Ansprüche, **dadurch gekennzeichnet, dass** die Zubereitung Ethanol enthält.

7. Kosmetische O/W-Emulsion nach einem der vorhergehenden Ansprüche, **dadurch gekennzeichnet, dass** die Zubereitung ein oder mehrere Antioxidantien enthält.

8. Kosmetische O/W-Emulsion nach einem der vorhergehenden Ansprüche, **dadurch gekennzeichnet, dass** die Zubereitung eine oder mehrere Verbindungen gewählt aus der Gruppe der Verbindungen Tocopherol, Tocopherolacetat, 2,6-Di-*tert*-butyl-4-methylphenol enthält.

9. Kosmetische O/W-Emulsion nach einem der vorhergehenden Ansprüche, **dadurch gekennzeichnet, dass** die Zubereitung ein oder mehrere Polymere enthält.

10. Kosmetische O/W-Emulsion nach einem der vorhergehenden Ansprüche, **dadurch gekennzeichnet, dass** die Zubereitung ein oder me hrere Polymere gewählt aus der Gruppe der Verbindungen Acrylat/C10-C30 Alkylacrylat Crosspolymer, Acrylate, Tapiokastärke, Xanthangummi enthält.

11. Kosmetische O/W-Emulsion nach einem der vorhergehenden Ansprüche, **dadurch gekennzeichnet, dass** die Zubereitung ein oder mehrere Ölkomponenten gewählt aus der Gruppe der Verbindungen Butylenglycol Dica prylat/Dicaprate, Myristylmyristat, Octyldodecanol, Mineralöl, Silikonöl enthält.

## Claims

1. Cosmetic O/W emulsion comprising
a) 2-ethylhexyl 2-cyano-3,3-diphenylacrylate (INCI: Octocrylene),
b) 4-(tert-butyl)-4'-methoxydibenzoylmethane (INCI: Butyl Methoxydibenzoylmethane),
c) licorice extract and
d) 2,4-bis{[4-(2-ethylhexyloxy)-2-hydroxy]phenyl}-6-(4-methoxyphenyl)-1,3,5-triazine (INCI: Aniso Triazine), **characterized in that** glyceryl stearate citrate, glyceryl stearate SE, stearic acid, polyglyceryl-3 methylglucose distearate, PEG-40 stearate, PEG-100 stearate, potassium cetyl phosphate and/or a mixture of cetearyl alcohol + PEG-40 hydrogenated castor oil + sodium cetearyl sulfate + glyceryl stearate is used as O/W emulsifier.

2. Cosmetic O/W emulsion comprising
a) 2-ethylhexyl 2-cyano-3,3-diphenylacrylate (INCI: Octocrylene),
b) 4-(tert-butyl)-4'-methoxydibenzoylmethane (INCI: Butyl Methoxydibenzoylmethane),
c) licochalcone A, and
d) 2,4-bis{[4-(2-ethylhexyloxy)-2-hydroxy]phenyl}-6-(4-methoxyphenyl)-1,3,5-triazine (INCI: Aniso Triazine).

3. Cosmetic O/W emulsion according to either of the preceding claims, **characterized in that** the preparation comprises at least 2.1% by weight, based on the total weight of the preparation, of 4-(tert-butyl)-4'-methoxydibenzoylmethane.

4. Cosmetic O/W emulsion according to Claim 2, **characterized in that** glyceryl stearate citrate, glyceryl stearate SE, stearic acid, polyglyceryl-3 methylglucose distearate, PEG-40 stearate, PEG-100 stearate, potassium cetyl phosphate and/or a mixture of cetearyl alcohol + PEG-40 hydrogenated castor oil + sodium cetearyl sulfate + glyceryl stearate is used as O/W emulsifier.

5. Cosmetic O/W emulsion according to any of the preceding claims, **characterized in that** the preparation comprises one or more UV filters selected from the group of compounds comprising phenylene-1,4-bis(2-benzimidazyl)-3,3'-5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylene-bis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate, homomenthyl salicylate; 2-ethylhexyl 2-hydroxybenzoate; dimethicodiethylbenzalmalonate; 3-(4-(2,2-bis ethoxycarbonylvinyl)phenoxy)propenyl)methoxysiloxane/ dimethylsiloxane copolymer; dioctylbutylamidotriazone (INCI: Diethylhexyl Butamidotriazone); 2,4-bis-[5-1(dimethylpropyl)benzoxazol-2-yl-(4-phenyl)imino]-6-(2-ethylhexyl)imino-1,3,5-triazine with the (CAS no. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis-(4'-dineopentylaminobenzalmalonate)-6-(4"-butylamino benzoate)-s-triazine, titanium dioxide; zinc oxide, trisbiphenyl-triazine 4-dicyanomethylene-2,6-dimethyl-1,4-dihydropyridine N-(ethyloxysulfate ester salt) mono-sodium salt.

6. Cosmetic O/W emulsion according to any of the preceding claims, **characterized in that** the preparation comprises ethanol.

7. Cosmetic O/W emulsion according to any of the preceding claims, **characterized in that** the preparation comprises one or more antioxidants.

8. Cosmetic O/W emulsion according to any of the preceding claims, **characterized in that** the preparation comprises one or more compounds selected from the group of compounds comprising tocopherol, tocopherol acetate, 2,6-di-*tert*-butyl-4-methylphenol.

9. Cosmetic O/W emulsion according to any of the preceding claims, **characterized in that** the preparation comprises one or more polymers.

10. Cosmetic O/W emulsion according to any of the preceding claims, **characterized in that** the preparation comprises one or more polymers selected from the group of compounds comprising acrylate/C10-C30 alkyl acrylate cross polymer, acrylates, tapioca starch, xanthan gum.

11. Cosmetic O/W emulsion according to any of the preceding claims, **characterized in that** the preparation comprises one or more oil components selected from the group of compounds comprising butylene glycol dicaprylate/dicaprate, myristyl myristate, octyldodecanol, mineral oil, silicone oil.

## Revendications

1. Émulsion H/E cosmétique contenant :
a) du 2-cyano-3,3-diphénylacrylate de 2-éthylhexyle (INCI : Octocrylene),
b) du 4-(tert.-butyl)-4'-méthoxydibenzoylméthane (INCI : Butylmethoxydibenzoylmethan),
c) de l'extrait de réglisse et
d) de la 2,4-bis-{[4-(2-éthyl-hexyloxy)-2-hydroxy]-phényl}-6-(4-méthoxyphényl)-1,3,5-triazine (INCI : Aniso Triazin), **caractérisée** en ce en ce qu'on utilise comme émulsifiant H/E le citrate-stéarate de glycéryle, le stéarate de glycéryle SE, l'acide stéarique, le distéarate de polyglycéryl-3-méthylglucose, le stéarate de PEG-40, le stéarate de PEG―100, le cétylphosphate de potassium et/ou un mélange d'alcool cétéarylique + PEG-40 huile de ricin hydrogénée + cétéarylsulfate de sodium + stéarate de glycéryle.

2. Émulsion H/E cosmétique contenant
a) du 2-cyano-3,3-diphénylacrylate de 2-éthylhexyle (INCI : Octocrylene),
b) du 4-(tert.-butyl)-4'-méthoxydibenzoylméthane (INCI : Butylmethoxydibenzoylmethan),
c) de la licochalcone A et
d) de la 2,4-bis-{[4-(2-éthyl-hexyloxy)-2-hydroxy]-phényl}-6-(4-méthoxyphényl)-1,3,5-triazine (INCI : Aniso Triazin).

3. Émulsion H/E cosmétique selon l'une quelconque des revendications précédentes, **caractérisée en ce que** la préparation contient au moins 2,1 % en poids, par rapport au poids total de la préparation, de 4-(tert.-butyl)-4'-méthoxydibenzoylméthane.

4. Émulsion H/E cosmétique selon l'une quelconque des revendications précédentes, **caractérisée en ce qu'**on utilise comme émulsifiant H/E le citrate-stéarate de glycéryle, le stéarate de glycéryle SE, l'acide stéarique, le distéarate de polyglycéryl-3-méthylglucose, le stéarate de PEG-40, le stéarate de PEG-100, le cétylphosphate de potassium et/ou un mélange d'alcool cétéarylique + PEG-40 huile de ricin hydrogénée + cétéarylsulfate de sodium + stéarate de glycéryle.

5. Émulsion H/E cosmétique selon l'une quelconque des revendications précédentes, **caractérisée en ce que** la préparation contient un ou plusieurs filtres UV choisis dans le groupe des composés sels d'acide phénylène-1,4-bis-(2-benzimidazyl)-3,3'-5,5'-tétrasulfonique ; sels d'acide 2-phénylbenzimidazole-5-sulfonique ; 1,4-di(2-oxo-10-sulfo-3-bornylidèneméthyl)-benzène et ses sels ; sels d'acide 4-(2-oxo-3-bornylidèneméthyl)benzènesulfonique ; sels d'acide 2-méthyl-5-(2-oxo-3-bornylidèneméthyl)sulfonique ; 2,2'-méthylène-bis(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphosulfonique ; 4-(diméthylamino)benzoate de 2-éthylhexyle ; 4-(diméthylamino)benzoate d'amyle ; 4-méthoxybenzalmalonate de di(2-éthylhexyle) ; 4-méthoxycinnamate de 2-éthylhexyle ; 4-méthoxycinnamate d'isoamyle ; 2-hydroxy-4-méthoxybenzophénone, 2-hydroxy-4-méthoxy-4'-méthylbenzophénone ; 2,2'-dihydroxy-4-méthoxybenzophénone ; 2-(4'-diéthylamino-2'-hydroxybenzoyl)-benzoate d'hexyle ; salicylate d'homomenthyle ; 2-hydroxybenzoate de 2-éthylhexyle ; benzalmalonate de diméthicodiéthyle ; copolymère de 3-(4-(2,2-biséthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine portant le (n° CAS 288254-16-0) ; 4,4',4"-(1,3,5-triazine-2,4,6-triyltriimino)-tris-benzoate de tris(2-éthylhexyle) (également : 2,4,6-tris[anilino-(p-carbo-2'-éthyl-1'-hexyloxy)]-1,3,5-triazine (INCI : Ethylhexyl Triazone) ; 2,4-bis-(4'-di-néopentylaminobenzalmalonate)-6-(4"-butylaminobenzoate)-s-triazine, dioxyde de titane, oxyde de zinc, trisbiphényl-triazine, sel monosodique de 4-dicyanométhylène-2,6-diéthyl-1,4-dihydropyridine-N-(sel d'ester d'éthyloxysulfate).

6. Émulsion H/E cosmétique selon l'une quelconque des revendications précédentes, **caractérisée en ce que** la préparation contient de l'éthanol.

7. Émulsion H/E cosmétique selon l'une quelconque des revendications précédentes, **caractérisée en ce que** la préparation contient un ou plusieurs antioxydants.

8. Émulsion H/E cosmétique selon l'une quelconque des revendications précédentes, **caractérisée en ce que** la préparation contient un ou plusieurs composés choisis dans le groupe des composés tocophérol, acétate de tocophérol, 2,6-di-*tert*-butyl-4-méthylphénol.

9. Émulsion H/E cosmétique selon l'une quelconque des revendications précédentes, **caractérisée en ce que** la préparation contient un ou plusieurs polymères.

10. Émulsion H/E cosmétique selon l'une quelconque des revendications précédentes, **caractérisée en ce que** la préparation contient un ou plusieurs polymères choisis dans le groupe des composés polymère croisé d'acrylate/acrylate d'alkyle en C10-C30, acrylates, amidon de tapioca, gomme xanthane.

11. Émulsion H/E cosmétique selon l'une quelconque des revendications précédentes, **caractérisée en ce que** la préparation contient un ou plusieurs composants huileux choisis dans le groupe des composés dicaprylate/dicaprate de butylèneglycol, myristate de myristyle, octyldodécanol, huile minérale, huile de silicone.
